# EUROPÄISCHE PATENTSCHRIFT

(11) **EP 0 888 103 B1**
(45) Veröffentlichungstag und Bekanntmachung des Hinweises auf die Patenterteilung: **19.06.2002**
(21) Anmeldenummer: 97906183.5
(22) Anmeldetag: 06.03.1997
(51) Int. Cl.: A61F 13/46

(54) **ABSORBIERENDER ARTIKEL ZUR GERICHTETEN ABFÜHRUNG VON LOKALISIERT AUSTRETENDEN FLUIDEN**
ABSORBENT ARTICLE FOR THE DIRECTED DRAINAGE OF FLUIDS EMERGING IN A LOCALIZED MANNER
ARTICLE ABSORBANT D'EVACUATION ORIENTEE DE FLUIDES SORTANT DE MANIERE LOCALISEE

(30) Priorität: 11.03.1996 DE 19609462
(43) Veröffentlichungstag der Anmeldung: 07.01.1999
(60) Teilanmeldung: 01117044.6
(73) Patentinhaber: Kimberly-Clark GmbH, 56070 Koblenz (DE)
(72) Erfinder: RAIDEL, Maria, D-90451 Nürnberg (DE); ASCHENBRENNER, Franz, D-92280 Kastl (DE)
(74) Vertreter: Diehl, Hermann, Dr. Dipl.-Phys.
(86) Internationale Anmeldenummer: EP9701144
(87) Internationale Veröffentlichungsnummer: WO9733546

(56) Entgegenhaltungen:
- EP-A- 0 321 985
- EP-A- 0 523 683
- EP-A- 0 596 532
- GB-A- 2 235 878
- US-A- 3 945 386

## Beschreibung

Die vorliegende Erfindung betrifft einen absorbierenden Artikel sowie ein Verfahren zur gerichteten Abführung von lokalisiert austretenden Fluiden.

Absorbierende Artikel sind als Hygieneprodukte seit langem bekannt. Sie finden beispielsweise als Windeln, Inkontinenzeinlagen oder Damenbinden Verwendung. Diese absorbierenden Artikel sind so ausgelegt, daß sie flüssige Körperausscheidungen, wie Urin, Menstruationsflüssigkeit oder Blut, aufnehmen und speichern können. Damenbinden werden beispielsweise eingesetzt, um die vor, während und nach der Menstruation ausgeschiedenen Flüssigkeiten zu absorbieren. Damenbinden werden außen (extern) am Körper getragen und unterscheiden sich insofern von Tampons, welche in die weibliche Vagina eingeführt werden und somit als "interne" Produkte bezeichnet werden können.

Als nachteilig wird beim Gebrauch von bekannten absorbierenden Artikeln häufig empfunden, daß die dem Körper zugewandte Oberfläche nach Beaufschlagung mit einer Flüssigkeit nasse Bereiche aufweist, was beim Träger zu einem unangenehmen Gefühl führt. Untersuchungen in diesem Zusammenhang haben ergeben, daß herkömmliche Damenbinden bereits rücknässen, wenn erst ca. 5 % des theoretischen Flüssigkeitsaufnahmevermögens der Binde ausgeschöpft sind. Des weiteren hinterlassen die ausgeschiedenen Körperflüssigkeiten auf der Oberfläche des verwendeten absorbierenden Artikels häufig sichtbare Rückstände, was den Verwender des Artikels dazu verleitet, den absorbierenden Artikel häufiger auszutauschen als dies von der Aufnahmefähigkeit für Flüssigkeiten her notwendig wäre.

Aus der EP 0 523 683 A1 ist ein absorbierender Artikel bekannt, der eine Zweikomponentenabdeckschicht aufweist. Die zwei Komponenten sind ein erstes Material mit darin ausgebildeten Öffnungen und ein zweites Material ohne Öffnungen. Beide Materialien wirken zur Umschließung eines absorbierenden Materials zusammen. Weiterhin ist eine Flußkontrollkomponente vorgesehen, die das erste Material von dieser ersten Oberfläche des absorbierenden Materials trennt und die Flüssigkeit in z-Richtung sowie in x- und y-Richtung leitet.

Es ist somit die Aufgabe der vorliegenden Erfindung, einen absorbierenden Artikel und ein Verfahren zur gerichteten Abführung von lokalisiert austretenden Flüssigkeiten zur Verfügung zu stellen, bei dem die Aufnahmefähigkeit des Flüssigkeitsspeichermaterials des absorbierenden Artikels optimal genützt wird und wobei auch nach längerem Gebrauch des absorbierenden Artikels auf der dem Körper zugewandten Seite möglichst wenig Gebrauchsspuren sichtbar sind.

Diese Aufgabe löst die vorliegende Erfindung durch den absorbierenden Artikel gemäß dem unabhängigen Patentanspruch 1
Weitere vorteilhafte Ausgestaltungen des erfindungsgemäßen absorbierenden Artikels und des erfindungsgemäßen Verfahrens ergeben sich aus den abhängigen Patentansprüchen, der Beschreibung und den Zeichnungen.

Der erfindungsgemäße absorbierende Artikel weist einen vorderen und einen hinteren Bereich auf, welche durch einen Mittelteil miteinander verbunden sind. Die bei Gebrauch des Artikels dem Körper zugewandte Schicht wird durch ein flüssigkeitsdurchlässiges Material gebildet, wohingegen die körperabgewandte Schicht des Artikels durch eine flüssigkeitsundurchlässige Schicht dargestellt wird. Zwischen der körperzugewandten, flüssigkeitsdurchlässigen Schicht und der körperabgewandten, flüssigkeitsundurchlässigen Schicht befindet sich eine Schicht, welche die in den absorbierenden Artikel eintretenden Fluide mit einer Vorzugsrichtung in dem Artikel verteilen kann. Diese wird im folgenden als Flüssigkeitsverteilungsschicht bezeichnet und erstreckt sich vom vorderen bis zum hinteren Bereich des absorbierenden Artikels. Des weiteren weist
der absorbierende Artikel gemäß der vorliegenden Erfindung eine Schicht auf, in welcher die eingetretene Flüssigkeit aufgenommen und zurückgehalten wird (sog. Flüssigkeitsspeicherschicht, auch als Saugkörper bezeichnet).

Der Artikel ist dadurch gekennzeichnet, dass die Flüssigkeitsverteilungsschicht den direkten Durchtritt von Flüssigkeit in die Flüssigkeitsspeicherschicht verhindert und einen gerichteten Transport der Flüssigkeit zu vorderen und/oder hinteren Bereichen des absorbierenden Artikels bewirkt, um dadurch einen Flüssigkeitstransport längs der Flüssigkeitsverteilungsschicht zu den vorderen und/oder hinteren Bereichen und danach in die Flüssigkeitsspeicherschicht in den jeweiligen vorderen und/oder hinteren Bereichen zu erreichen, wodurch die Überführung der Flüssigkeit von der Flüssigkeitsverteilungsschicht zu der Flüssigkeitsspeicherschicht hauptsächlich in den jeweiligen vorderen und/oder hinteren Bereichen der Flüssigkeitsspeicherschicht erfolgt.

Durch den spezifischen Aufbau des erfindungsgemäßen absorbierenden Artikels wird erreicht, daß die Speicherung einer eingedrungenen Flüssigkeit bevorzugt in den Endbereichen des Artikels erfolgt. Außerdem erfolgt die Speicherung der Flüssigkeit in den unteren Schichten leichter, wodurch sich ein sog. "bottom-up-filling"-Effekt ergibt.

Vorzugsweise ist die Flüssigkeitsverteilungsschicht so ausgestaltet, daß die in den absorbierenden Körper eingedrungene Flüssigkeit von der zentral im Mittelteil gelegenen Beaufschlagungsstelle in den vorderen und hinteren Bereich geleitet wird. Im Falle eines länglich ausgestalteten absorbierenden Artikels wird somit eine Flüssigkeitsverteilung in Längsrichtung des Artikels bewirkt.

Die Flüssigkeitsverteilungsschicht kann sich dabei über die gesamte Breite des absorbierenden Artikels bzw. der Flüssigkeitsspeicher erstrecken. Bei Verwendung eines leistungsfähigen Flüssigkeitstransportsystems innerhalb des absorbierenden Artikels kann es aber auch ausreichend sein, wenn die Breite der Flüssigkeitsverteilungsschicht kleiner ist als die geringste Breite des absorbierenden Artikels bzw. der flüssigkeitsspeichernden Schicht.

Die Flüssigkeitsverteilung (Drainage) zu dem vorderen und hinteren Ende des Artikels wird besonders günstig dadurch erreicht, indem die Flüssigkeitsverteilungsschicht eine gewellte bzw. plissierte Bahn aufweist, deren Wellen so angeordnet sind, daß die Flüssigkeit bevorzugt in Längsrichtung zu den Enden des Artikels hin abgeleitet wird. Die gewellte bzw. plissierte Bahn kann z.B. aus einem Vliesmaterial bestehen, welches praktisch keine eigene Saugkapazität aufweist, wodurch verhindert wird, daß diese Bahn Flüssigkeit dauerhaft zurückhält. Die gewellte Bahn dient vornehmlich der Flüssikeitsleitung (Drainage) und als Abstandshalter. Indem die gewellte Bahn außerdem Pigmentstoffe enthält, kann ein Durchscheinen des mit Flüssigkeit beaufschlagten Saugkörpers verhindert werden, wodurch das subjektive Sauberkeitsgefühl des Trägers bzw. der Trägerin des absorbierenden Artikels erhöht wird.

Die gewellte Bahn ist vorzugsweise mit einer weiteren Bahn verbunden, die einerseits die Stabilisierung der Wellung und andererseits den gerichteten Transport der in den Artikel eingedrungenen Flüssigkeit unterstützen kann. Als besonders geeignetes Material für eine solche weitere Bahn hat sich ein sog. "uncreped through air dried"-Material (UCTAD-Material) erwiesen.

Ein bevorzugtes UCTAD-Material enthält mindestens 10 Gew.-% hochergiebige Zellstoffasern (high yield pulp fibers), bezogen auf die Trockensubstanz, zu denen ein Naßfestmittel in einer Menge zugegeben wird, die bewirkt, daß das Verhältnis der Naßreißfestigkeit zu der Trockenreißfestigkeit mindestens etwa 0,1 beträgt. Hochergiebige Zellstoffasern enthalten viel Lignin, auf welches die Naßelastizität der Fasern zurückzuführen sein dürfte. Die durch das Naßfestmittel ausgebildeten Harzbindungen immobilisieren die naßelastischen Fasern in einer blattartigen Struktur, welche sich der Struktur des Bandes anpaßt, auf dem die Trocknung (throughdrying) erfolgt. Während des Trocknungsvorgangs werden die von dem Naßfestmittel gebildeten Bindungen ausgehärtet, so daß sich nässeresistente Bindungen ausbilden, was wiederum die hochelastischen Eigenschaften einer entsprechenden Bahn in nassem Zustand bewirkt. Diese Eigenschaft behält die Bahn bei, da bei einem UCTAD-Verfahren kein Kreppschritt oder anderer Schritt, welcher die Bindungen wieder zerstören könnte, durchgeführt wird. Somit ist das UCTAD-Material hervorragend geeignet, Flüssigkeiten zu transportieren, da das Material auch in nassem Zustand stabilisiert ist.

Eine weitere Ausführungsform der flüssigkeitsverteilenden Schicht des erfindungsgemäßen absorbierenden Artikels ist so ausgestaltet, daß die gewellte Bahn auf einem sich über die gesamte Breite des absorbierenden Artikels erstreckenden, flüssigkeitsdurchlässigen Trägermaterial plissiert ist, wobei die gewellte Bahn nur einen Teil der Gesamtbreite des Trägermaterials einzunehmen braucht. Unterhalb der gewellten Bahn mit Träger kann wieder die weitere Bahn angeordnet sein. Als Materialien für die gewellte Bahn bzw. die weitere Bahn sind die vorgenannten Materialien Spinnvlies bzw. UCTAD geeignet. Als Trägermaterial bietet sich ebenfalls Spinnvlies an.

Ein weiteres wichtiges Element des erfindungsgemäßen absorbierenden Artikels ist die Überführung eines Fluids von der Flüssigkeitsverteilungsschicht, wenigstens in den im vorderen Bereich und/oder hinteren Bereich des absorbierenden Artikels gelegenen Teil der Flüssigkeitsspeicherschicht. Diese Überführung kann beispielsweise durch Bereiche der Flüssigkeitsverteilungsschicht bewirkt werden,
die durch Pressen mit der Flüssigkeitsspeicherschicht in engen Kontakt gebracht worden sind; beispielsweise durch ein Kalandrierverfahren. Vorteilhafterweise sind diese Bereiche, welche durch Pressen miteinander in Kontakt gebracht worden sind, punktförmig ausgestaltet.

Der Flüssigkeitstransport von der Flüssigkeitsverteilungsschicht in die Flüssigkeitsspeicherschicht kann weiterhin dadurch unterstützt werden, indem die beiden genannten Schichten durch ein Haftmittel miteinander in Verbindung gebracht sind. Als besonders günstig hat es sich erwiesen, wenn das Haftmittel ein hydrophiler Klebstoff ist. Auch die Verbindung der Flüssigkeitsverteilungsschicht und der Flüssigkeitsspeicherschicht mittels des Haftmittels erfolgt vorzugsweise punktförmig. Auch geometrische Muster sind bevorzugt, wobei ein rautenförmiges Muster einen besonders effektiven Flüssigkeitstransfer von der Flüssigkeitsverteilungsschicht in die Flüssigkeitsspeicherschicht bewirkt.

Gemäß einer weiteren bevorzugten Ausführungsform des erfindungsgemäßen absorbierenden Artikels wird die Überführung eines Fluids von der Flüssigkeitsverteilungsschicht in die Flüssigkeitsspeicherschicht dadurch bewirkt, daß die Flüssigkeitsverteilungsschicht trichterförmige Öffnungen aufweist, die so ausgerichtet sind, daß sich die Trichteröffnungen in Richtung auf die Flüssigkeitsspeicherschicht hin verjüngen. Durch die trichterförmigen Öffnungen ist ein gerichteter Abfluß der Flüssigkeit aus der Flüssigkeitsverteilungsschicht in die Flüssigkeitsspeicherschicht gewährleistet. Des weiteren bewirken die trichterförmigen Öffnungen, daß die Flüssigkeitsverteilungsschicht von der Flüssigkeitsspeicherschicht beabstandet bleibt, wodurch ein Rücktransport der Flüssigkeit aus der Flüssigkeitsspeicherschicht in die Flüssigkeitsverteilungsschicht wirksam verhindert wird. Die trichterförmigen Öffnungen finden sich dabei vorzugsweise in der weiteren Bahn, welche mit der plissierten Bahn verbunden ist.

Gemäß einer bevorzugten Ausführungsform des erfindungsgemäßen Artikels weist die weitere Bahn einen in Längsrichtung gesehen mittleren Bereich ohne trichterförmige Öffnungen auf, auf welchen die gewellte Bahn aufgebracht ist. In den in Längsrichtung gesehen randseitig gelegenen Bereich befinden sich die trichterförmigen Öffnungen. Die Randbereiche mit den trichterförmigen Öffnungen werden nun derart unter den mit der gewellten Bahn versehenen Bereich der weiteren Bahn geklappt, daß die trichterförmigen Öffnungen mit ihren sich verjüngenden Bereichen der Flüssigkeitsspeicherschicht gegenüberliegen. Durch das Einbringen der trichterförmigen Öffnungen in die weitere Bahn, beispielsweise mittels Nadeln, wird die weitere Bahn durchstochen, wodurch sich am verjüngenden Ende der trichterförmigen Öffnungen kleine Saugfüßchen ausbilden können. Ein besonders effektiver Fluidtransport wird dann erreicht, wenn diese Saugfüßchen mit der Flüssigkeitsspeicherschicht in Eingriff stehen, da dies den gerichteten Transport der Flüssigkeit von der Flüssigkeitsverteilungsschicht in die Flüssigkeitsspeicherschicht unterstützt. Das Einklappen der weiteren Bahn kann so ausgestaltet sein, daß sie von jeder Seite bis etwa zur Mitte der weiteren Bahn erfolgt. Die Randbereiche können aber auch die selbe Breite aufweisen wie der Bereich der weiteren Bahn, auf dem die gewellte Bahn plissiert ist. Durch Einklappen der mit trichterförmigen Öffnungen versehenen Randbereiche unter die weitere Bahn ergibt sich dann eine Dreifachschichtstruktur. Diese Dreifachschichtstruktur verhindert besonders wirksam das Rücknässen von Flüssigkeit aus dem Flüssigkeitsspeicher in die Flüssigkeitsverteilungsschicht.

Ein gerichteter Flüssigkeitstransport aus der Flüssigkeitsverteilungsschicht in die Flüssigkeitsspeicherschicht, beziehungsweise den Saugkörper, wird auch dadurch unterstützt, indem die Flüssigkeitsspeicherschicht Bereiche höheren Flüssigkeitsrückhaltevermögens aufweist, wobei in diese Bereiche Flüssigkeit bevorzugt abgegeben wird. Wenn die Flüssigkeitsspeicherschicht aus einem Zellstoff aufgebaut ist, kann das höhere Rückhaltevermögen durch eine Verdichtung des Zellstoffmaterials bewirkt werden. Durch die Verdichtung verringern sich die Poren bzw. die Porengröße in dem Zellstoffmaterial, was die Kapillarkräfte erhöht. Eine weitere Möglichkeit, in dem Saugkörper lokal begrenzte Bereiche höheren Flüssigkeitsrückhaltevermögens auszubilden, besteht darin, superabsorbierende Materialien in diesen lokal begrenzten Bereichen einzusetzen.

Wenn, wie im vorliegenden Fall, eine optimale Ausnützung des Saugkörpers zur Flüssigkeitsspeicherung gewünscht wird, ist es von Vorteil, wenn zunächst die endständigen Bereiche des absorbierenden Artikels die Flüssigkeit speichern und eine Flüssigkeitsspeicherung im Mittelteil des Artikels erst dann erfolgt, wenn die Saugkapazitäten der Endbereiche erschöpft sind. Dadurch kann die Saugkörperkapazität optimal ausgenützt werden, wodurch der Träger bzw. die Trägerin des absorbierenden Artikels diesen nicht so häufig wechseln muß.

Diese gerichtete Auffüllung des Saugkörpers mit Flüssigkeit kann des weiteren auch dadurch unterstützt werden, indem die weitere Bahn im Mittelteil des absorbierenden Artikels keine trichterförmigen Öffnungen aufweist und die trichterförmigen Öffnungen nur im vorderen und/oder im hinteren Bereich des absorbierenden Artikels angeordnet sind. Dadurch wird verhindert, daß bei Beaufschlagung des absorbierenden Artikels im Mittelteil die in den Artikel eintretende Flüssigkeit direkt in dem unter dem Eintrittsbereich liegenden Bereich der Flüssigkeitsspeicherschicht abgelagert wird. Dies ist weniger wünschenswert, da dann die Saugkapazität des absorbierenden Artikels nicht voll ausgeschöpft werden könnte, bevor eine Rücknässung auf die dem Körper zugewandte Oberfläche des Artikels erfolgt.

Die Gefahr der Rücknässung kann des weiteren dadurch minimiert werden, wenn zwischen dem seitlichen Rand des absorbierenden Artikels und der Flüssigkeitsverteilungsschicht eine flüssigkeitsundurchlässige Sperrschicht angeordnet ist. Dies empfiehlt sich besonders dann, wenn die Breite b der Flüssigkeitsverteilungsschicht kleiner ist als die geringste Breite B der Flüssigkeitsspeicherschicht. Diese Sperrschicht bewirkt, daß aus der Flüssigkeitsspeicherschicht austretende Flüssigkeit nicht an der Flüssigkeitsverteilungsschicht vorbei zur Oberfläche des absorbierenden Artikels gelangen kann, wodurch der Tragekomfort des absorbierenden Artikels weiter erhöht wird.

Weiterhin kann der erfindungsgemäße absorbierende Artikel zwischen der flüssigkeitsdurchlässigen Schicht und der Flüssigkeitsverteilungsschicht eine flüssigkeitsaufnehmende Schicht enthalten. Diese flüssigkeitsaufnehmende Schicht ist nicht in erster Linie dazu vorgesehen, als Primärspeicher oder Sekundärspeicher zu dienen. Der Zweck der flüssigkeitsaufnehmenden Schicht ist vielmehr darin zu sehen, daß Flüssigkeit, die aus der Flüssigkeitsspeicherschicht wieder ausgetreten ist bzw. erst gar nicht in den Flüssigkeitsspeicher eintreten konnte, vor einem Austreten auf die Oberfläche des absorbierenden Artikels abgefangen wird. Die flüssigkeitsaufnehmende Schicht ist somit als eine Speichereinrichtung anzusehen, die im Falle einer Überforderung der Flüssigkeitsspeicherschicht in der Lage ist, Flüssigkeiten aufzunehmen und zu speichern, bevor diese zu einer Rücknässung auf der Oberfläche des absorbierenden Artikels führen. Um das Eindringen der Flüssigkeiten in den absorbierenden Körper und die Verteilung der Flüssigkeit mittels der Flüssigkeitsverteilungsschicht nicht unnötig zu behindern, ist es bevorzugt, wenn die flüssigkeitsaufnehmende Schicht eine zentral gelegene, durch die gesamte Schicht hindurchreichende Öffnung aufweist. Diese Öffnung sollte so ausgestaltet sein, daß sie beim Tragen des absorbierenden Artikels der Körperöffnung gegenüberliegt, aus dem die aufzunehmende Flüssigkeit austritt. Die flüssigkeitsaufnehmende Schicht kann aus Zellstoffmaterial aufgebaut sein oder ein solches Zellstoffmaterial enthalten. Eine Zellstoffmaterialschicht ist weich und erhöht damit den Tragekomfort eines entsprechend aufgebauten absorbierenden Artikels.

Ein weiteres geeignetes Material für die flüssigkeitsaufnehmende Schicht ist eine Zellstoffmischung, wie beispielsweise sog. Coform-Material. Coform-Material besteht aus Zellstoff- und Polypropylenfasern in unterschiedlichsten Mischverhältnissen. Die Polypropylenfasern sind extrudiert. Die flüssigkeitsaufnehmende und die flüssigkeitsspeichernde Schicht können aus dem gleichen Material bestehen.

Die flüssigkeitsundurchlässige Sperrschicht und die flüssigkeitsundurchlässige Abdeckschicht des absorbierenden Artikels gemäß der Erfindung können aus Polyethylen, Polypropylen oder Mischungen dieser Polymere hergestellt sein. Um beim Tragen des absorbierenden Artikels dessen paßgenauen Sitz gewährleisten und ein Verrutschen zu verhindern, kann auf der Außenseite der flüssigkeitsundurchlässigen Abdeckschicht eine Haftschicht angebracht sein, mit welcher der absorbierende Artikel in einem Kleidungsstück befestigt werden kann.

Der gerichtete Flüssigkeitstransport in dem erfindungsgemäßen absorbierenden Artikel kann des weiteren durch zusätzliche Mittel, wie beispielsweise zusätzliche Bahnen mit bevorzugter Transportrichtung, unterstützt werden. Solche zusätzlichen Mittel sind günstigerweise zwischen der Flüssigkeitsverteilungsschicht und der Flüssigkeitsspeicherschicht angeordnet.

Die flüssigkeitsdurchlässige Abdeckschicht und die flüssigkeitsaufnehmende Schicht können leicht miteinander verbunden sein, beispielsweise durch punktuelles Pressen.

Der erfindungsgemäße absorbierende Artikel kann besonders als Damenbinde oder Damenhygieneeinlage Verwendung finden.

Durch die vorliegende Erfindung werden lokalisiert austretende Flüssigkeiten gerichtet abgeführt. Dabei wird ein absorbierender
Artikel mit einer Flüssigkeit beaufschlagt, wobei die Flüssigkeit durch eine flüssigkeitsdurchlässige Abdeckschicht in den absorbierenden Artikel eintritt. Die eintretende Flüssigkeit wird dann mittels einer Flüssigkeitsverteilungsschicht in die Bereiche des absorbierenden Artikels transferiert, in denen eine Flüssigkeitsspeicherung erwünscht ist. Die Überführung der Flüssigkeit von der Flüssigkeitsverteilungsschicht in die Flüssigkeitsspeicherschicht erfolgt durch Mittel, die einen gerichteten Transport der Flüssigkeit ermöglichen.

Die vorliegende Erfindung wird nachfolgend anhand der Zeichnungen und bevorzugter Ausführungsformen näher erläutert. In den Zeichnungen zeigen:
- Fig. 1: eine perspektivische Ansicht einer Ausführungsform des erfindungsgemäßen Artikels, teilweise im Schnitt;
- Fig. 2: einen funktionellen Längsschnitt durch den erfindungsgemäßen absorbierenden Artikel entlang der Linie 2-2 aus Fig. 1;
- Fig. 3A: einen vergrößerten Ausschnitt eines Bereichs aus Fig. 2;
- Fig. 3B: einen vergrößerten Ausschnitt eines Bereichs aus Fig. 2 gemäß einer weiteren erfindungsgemäßen Ausführungsform;
- Fig. 4: einen Querschnitt durch die in Fig. 1 gezeigte Ausführungsform des absorbierenden Artikels entlang der Linie 4-4 von Fig. 1;
- Fig. 5: einen vergrößert dargestellten Ausschnitt eines Bereichs aus Fig. 4;
- Fig. 6: einen funktionellen Längsschnitt durch eine weitere Ausführungsform des erfindungsgemäßen absorbierenden Artikels;
- Fig. 7: eine Detaildarstellung eines Teils der Flüssigkeitsverteilungsschicht einer Ausführungsform des erfindungsgemäßen absorbierenden Artikels;
- Fig. 8: den in Fig. 7 gezeigten Teil der Flüssigkeitsverteilungsschicht in zusammengefalteter, gebrauchsfertiger Form;
- Fig. 9: eine Detaildarstellung eines Teils einer Flüssigkeitsverteilungsschicht einer weiteren Ausführungsform des erfindungegemäßen absorbierenden Artikels;
- Fig. 10: den in Fig. 9 gezeigten Teil der Flüssigkeitsverteilungsschicht in zusammengefalteter, gebrauchsfertiger Form; und
- Fig. 11: eine perspektivische Ansicht einer weiteren Ausführungsform eines erfindungsgemäßen absorbierenden Artikels.

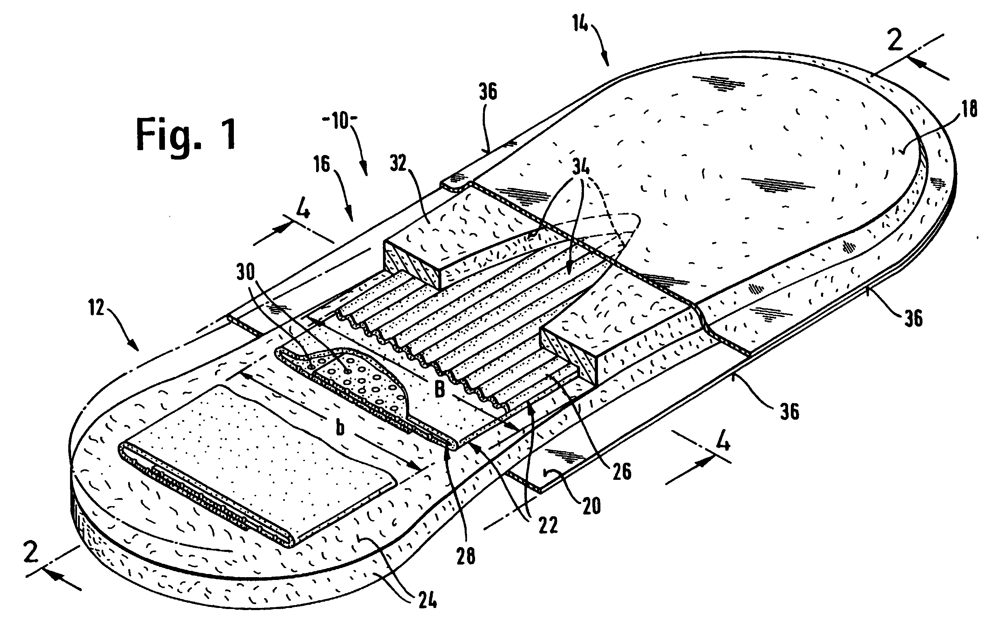

Fig. 1 zeigt einen absorbierenden Artikel 10 mit einem vorderen Bereich 12, einem hinteren Bereich 14 sowie einem den vorderen und den hinteren Bereich verbindenden Mittelteil 16. Mit dem Bezugszeichen 18 ist die flüssigkeitsdurchlässige obere Abdeckschicht aus hochpigmentiertem Spinnvlies, mit dem Bezugszeichen 20 die untere flüssigkeitsundurchlässige Schicht des absorbierenden Artikels bezeichnet. Unterhalb der flüssigkeitsdurchlässigen Schicht 18 ist die Flüssigkeitsverteilungsschicht 22 angeordnet. Der gerichtete Transport von Flüssigkeiten in die Endbereiche des absorbierenden Artikels 10 erfolgt bei der Ausführungsform gemäß Fig. 1 mittels der gewellten Bahn 26. Die gewellte Bahn 26 ist auf eine weitere Bahn 28 plissiert. Die gewellte Bahn 26 ist aus einem Vliesstoffmaterial (pigmentiertes Spinnvlies) und die weitere Bahn 28 aus einem UCTAD-Material aufgebaut. Die weitere Bahn 28 ist dabei in den Randbereichen nach unten umgeschlagen, so daß die umgeschlagenen Bereiche parallel zur gewellten Bahn zu liegen kommen. In den umgeschlagenen Bereichen der weiteren Bahn 28 befinden sich trichterförmige Öffnungen 30. Unterhalb der weiteren Bahn 28 ist die Flüssigkeitsspeicherschicht 24 gelegen.

Gemäß einer weiteren bevorzugten Ausführungsform des erfindungsgemäßen absorbierenden Artikels kann dieser eine flüssigkeitsaufnehmende Schicht 32 enthalten. Diese flüssigkeitsaufnehmende Schicht 32 ist dazu vorgesehen, in dem Flüssigkeitsspeicher 24 aufgenommene und aus diesem wieder austretende, seitlich an der Flüssigkeitsverteilungsschicht 22 vorbeitretende Flüssigkeit abzufangen, bevor diese durch die flüssigkeitsdurchlässige Schicht 18 auf die Oberfläche des absorbierenden Artikels austreten kann. Besonders günstig ist es, wenn die flüssigkeitsaufnehmende Schicht 32 eine zentral gelegene, durchgehende Öffnung 34 aufweist. Die zentrale Öffnung 34 kommt bei Gebrauch des absorbierenden Artikels gegenüber der Körperöffnung des Trägers bzw. der Trägerin zum Liegen, aus der die aufzunehmende Flüssigkeit austritt. Die Öffnung 34 bewirkt, daß die aufzunehmende Flüssigkeit, bevor diese auf die Flüssigkeitsverteilungsschicht 22 gelangt, nicht durch ein absorbierendes Material durchtreten muß, was die gewünschte Verteilung der Flüssigkeit in Längsrichtung des absorbierenden Körpers unterstützt. Die flüssigkeitsdurchlässige Bahn 18 und die gewellte Bahn 26 sind aus einem Vliesstoff hergestellt, der Pigmente enthält. Dadurch wird erreicht, daß in der Flüssigkeitsspeicherschicht 24 festgehaltene Flüssigkeit nicht bis zur Oberfläche des absorbierenden Artikels durchscheinen kann. Dies erhöht die Verbraucherakzeptanz des absorbierenden Artikels. Auch nach einer Beaufschlagung des absorbierenden Artikels mit einer Flüssigkeit ist von außen kaum sichtbar, daß dieser bereits u.U. größere Mengen an Flüssigkeiten aufgenommen hat.

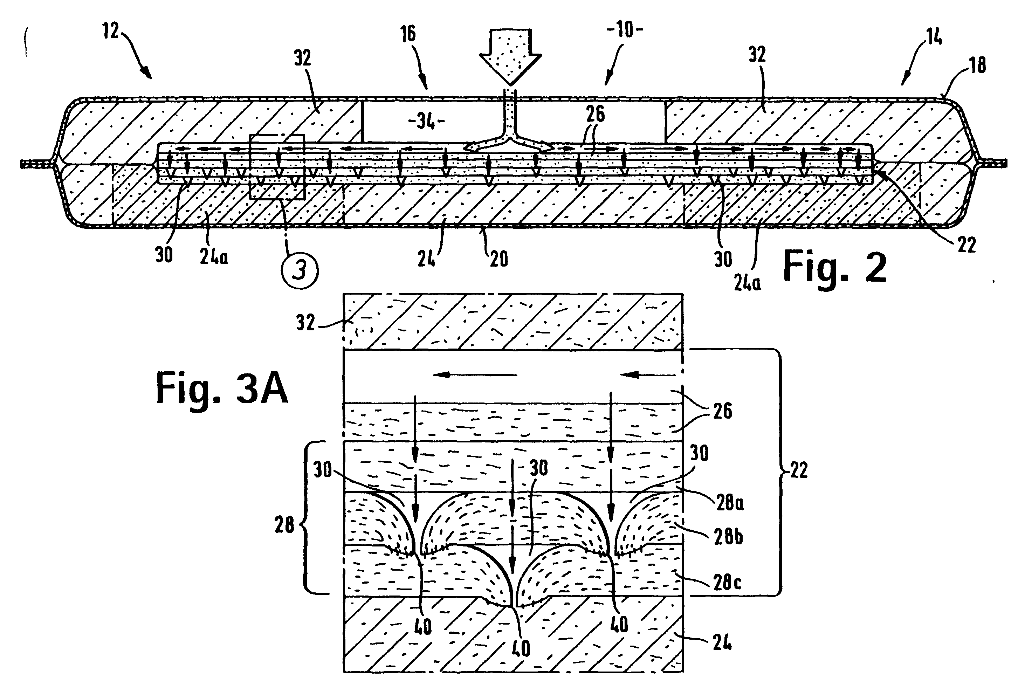

Fig. 2 ist ein funktioneller Längsschnitt durch den erfindungsgemäßen absorbierenden Artikel entlang der Linie 2 aus Fig. 1. Die Pfeile geben dabei die Flüssigkeitsströmung innerhalb des absorbierenden Artikels an. Die durch die flüssigkeitsdurchlässige Schicht 18 hindurchtretende Flüssigkeit trifft auf die Flüssigkeitsverteilungsschicht 22 auf. Die Flüssigkeitsverteilungsschicht 22 verhindert dabei, daß die Flüssigkeit direkt in die flüssigkeitsaufnehmende Schicht 24 durchtritt. Es erfolgt vielmehr eine gerichteter Transport in Richtung auf den vorderen Bereich 12 bzw. den hinteren Bereich 14 des absorbierenden Artikels. Der Flüssigkeitsübertritt aus der Flüssigkeitsverteilungsschicht 22 in die flüssigkeitsaufnehmende Schicht 24 erfolgt dabei bevorzugt in den Bereichen erhöhten Flüssigkeitsrückhaltevermögens 24a der Flüssigkeitsspeicherschicht 24. Die Speicherschicht 24 besteht aus Zellstoff. Das erhöhte Flüssigkeitsrückhaltevermögen der Bereiche 24a wird durch eine Verdichtung des Zellstoffmaterials erreicht, was durch die dichtere Schraffierung in Fig. 2 dargestellt ist. Ebenfalls dargestellt sind die in der Schicht 22 angeordneten trichterförmigen Öffnungen 30, die einen vertikalen Transport der Flüssigkeit von oben nach unten bewirken, wobei aufgrund der Struktur der trichterförmigen Öffnungen praktisch kein Rücktransport der Flüssigkeit aus tiefer liegenden Bereichen in höher liegende Bereiche erfolgt. Ebenfalls gezeigt in Fig. 2 ist die flüssigkeitsaufnehmende Schicht 32, welche nicht in den Flüssigkeitsspeicher 24 aufgenommene oder aus diesem wieder ausgetretene Flüssigkeiten abfangen kann, bevor diese durch die flüssigkeitsdurchlässige Schicht 18 wieder auf die Oberfläche des absorbierenden Artikels austreten können.

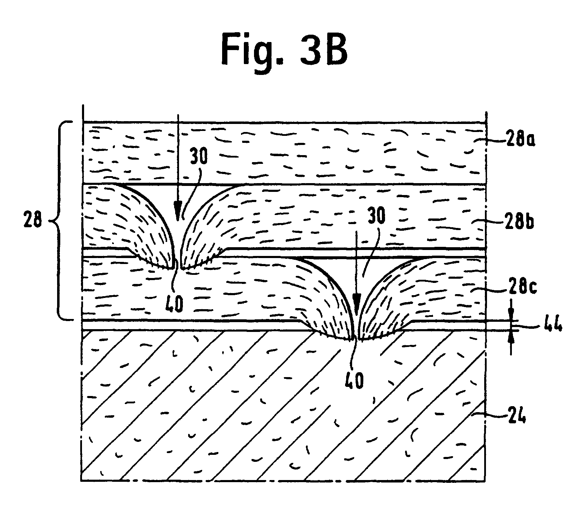

Fig. 3A ist ein vergrößerter Ausschnitt des Kontaktbereichs zwischen der Flüssigkeitsverteilungsschicht 22 und der flüssigkeitsspeichernden Schicht 24. Durch die Pfeile in Fig. 3 ist wiederum die bevorzugte Flüssigkeitstransportrichtung angegeben. Fig. 3 zeigt die dreilagig aufgebaute weitere Bahn 28. Die oberste Schicht 28a, auf welcher die gewellte Bahn aufgebracht ist, ist dabei ohne trichterförmige Öffnungen ausgestaltet. Die darunter liegenden Schichten 28b und 28c weisen dagegen die trichterförmigen Öffnungen 30 auf. Durch die Ausgestaltung der trichterförmigen Öffnungen 30 wird bewirkt, daß die Schichten 28b und 28c voneinander beabstandet sein können, wie dies in Fig. 3A gezeigt ist, wodurch ein Zurückschlagen der Flüssigkeit nach oben behindert wird. Zwischen der Schicht 28c und der weiteren Bahn der flüssigkeitsspeichernden Schicht 24 kann durch die trichterförmigen Öffnungen 30 ein Abstand 44 ausgebildet sein (Fig. 3B), welcher ebenfalls dazu beiträgt, zu verhindern, daß Flüssigkeit aus der flüssigkeitsspeichernden Schicht 24 wieder in die Flüssigkeitsverteilungsschicht 28 übertritt.

Ebenfalls gezeigt in den Fig. 3A und 3B sind die Saugfüßchen 40 am Ausgang der Trichteröffnungen 30. Die Saugfüßchen 40 haken sich jeweils in der darunterliegenden Bahn ein und unterstützen so einen gerichteten Flüssigkeitstransport auf die Flüssigkeitsspeicherschicht 24 hin.

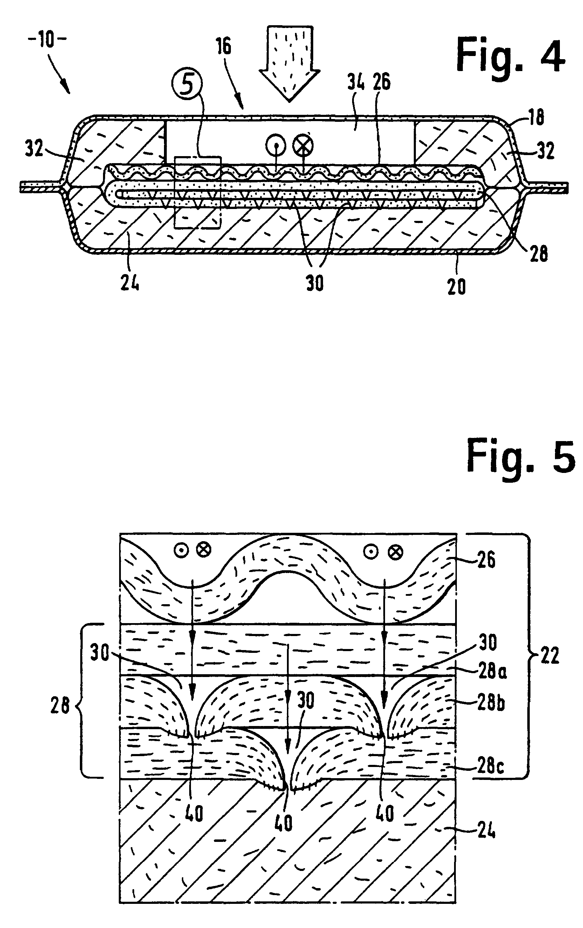

Fig. 4 zeigt einen Querschnitt durch den erfindungsgemäßen absorbierenden Artikel entlang der Linie 4-4 in Fig. 1. Der Pfeil zeigt die Richtung des Flüssigkeitseintritts in den absorbierenden Artikel an. Die eingetretene Flüssigkeit wird dann entlang der gewellten Bahn 26, die zu den Enden des absorbierenden Artikels 10 führt, transportiert. Des weiteren ist in Fig. 4 die weitere Bahn 28 im Querschnitt gezeigt. Der Teil der Bahn 28, welcher die gewellte Bahn 26 trägt, enthält keine Öffnungen. Die darunter angeordneten Seitenteile stehen mit der jeweils darunter befindlichen Schicht über Öffnungen 30 in Fluidkontakt. Des weiteren erstrecken sich die trichterförmigen Öffnungen 30 im untersten Bereich der Schicht 28 bis in die Flüssigkeitsspeicherschicht 24.

Fig. 5 ist ein vergrößert dargestellter Ausschnitt aus Fig. 4. Sie zeigt ebenfalls die gewellte Bahn 26 im Querschnitt. Die gewellte Bahn 26 ist über Kontaktpunkte mit der weiteren Bahn 28 verbunden. Die Schicht 28 besteht aus den drei Lagen 28a, 28b und 28c. Die die gewellte Bahn 26 tragende Schicht 28a weist keine Öffnungen 30 auf. Die beiden darunter angeordneten Schichten 28b und 28c dagegen enthalten die trichterförmigen Öffnungen 30, welche einen vertikal gerichteten Fluß der eingedrungenen Flüssigkeit unterstützen. Durch diesen nach unten gerichteten vertikalen Fluß wird eine Kanalstruktur geschaffen, welche ein Zurückschlagen der Flüssigkeit nach oben verhindert. Eine Flüssigkeitsabgabe durch die Schicht 28 nach oben findet praktisch nicht statt.

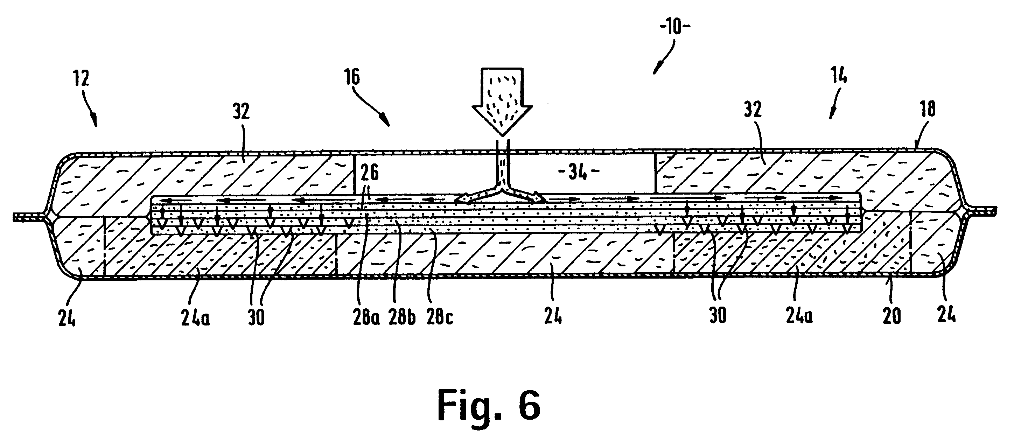

Fig. 6 zeigt einen funktionellen Längsschnitt durch eine weitere Ausführungsform des erfindungsgemäßen absorbierenden Artikels. Die Ausführungsform gemäß Fig. 6 weist die Besonderheit auf, daß die weitere Bahn 28 im mittleren Bereich des absorbierenden Artikels keine trichterförmigen Öffnungen 30 enthält. Dadurch wird erreicht, daß der Flüssigkeitstransport noch effektiver in die längsseitig gelegenen Endbereiche der Bahn 28 transportiert wird, bevor die Flüssigkeit in die Flüssigkeitsspeicherschicht 24 bzw. 24a abgegeben wird.

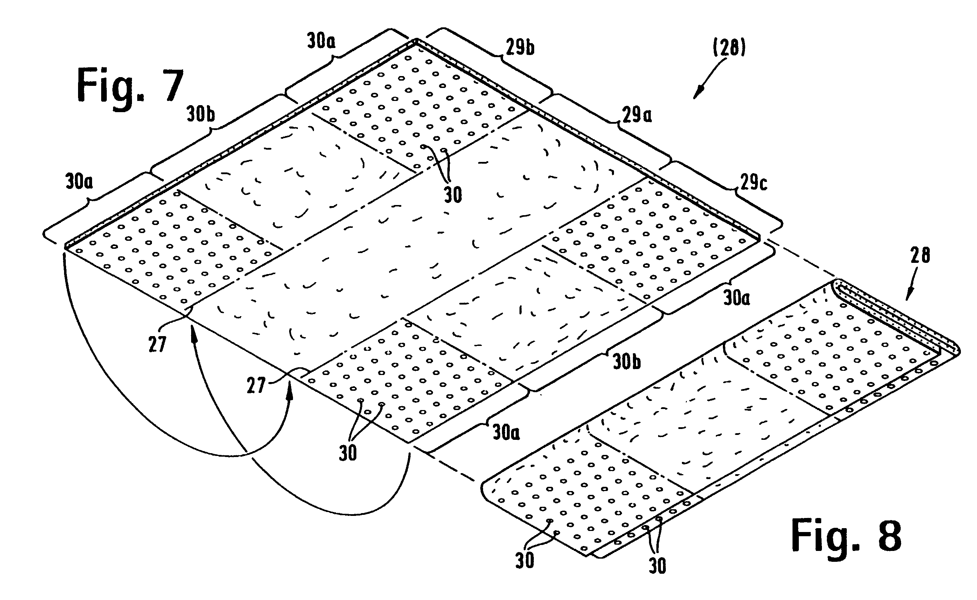

Fig. 7 zeigt die weitere Bahn 28. Diese weist, in Längsrichtung gesehen, eine zentrale, trichteröffnungsfreie Zone 29a auf. Die randseitig gelegenen Abschnitte 29b und 29c der weiteren Bahn 28 enthalten sowohl Bereiche 30a mit Öffnungen als auch einen Bereich 30b ohne Öffnungen. Beim Einsatz der weiteren Bahn 28 in dem erfindungsgemäßen absorbierenden Artikel 10 wird die Bahn 28 dann entlang den Linien 27 nach innen umgeklappt, wodurch die in Fig. 8 gezeigte Struktur erhalten wird. Die gefaltete Struktur der Bahn 28 enthält nun selektiv in den in Längsrichtung gesehenen Endbereichen Öffnungen 30, während der zentrale Teil völlig öffnungsfrei ist. Durch diese Konstruktion der weiteren Bahn 28 wird der vorstehend schon näher beschriebene gerichtete Fluß der in den absorbierenden Artikel eingedrungenen Flüssigkeit in den vorderen und hinteren Teil des absorbierenden Artikels bewirkt. Erst wenn sich die Flüssigkeitsspeicherschicht im vorderen und hinteren Endbereich des absorbierenden Artikels mit Flüssigkeit vollgesaugt hat, wird auch der im Mittelteil des Artikels gelegene Bereich der Flüssigkeitsspeicherschicht 24 mit Flüssigkeit gefüllt.

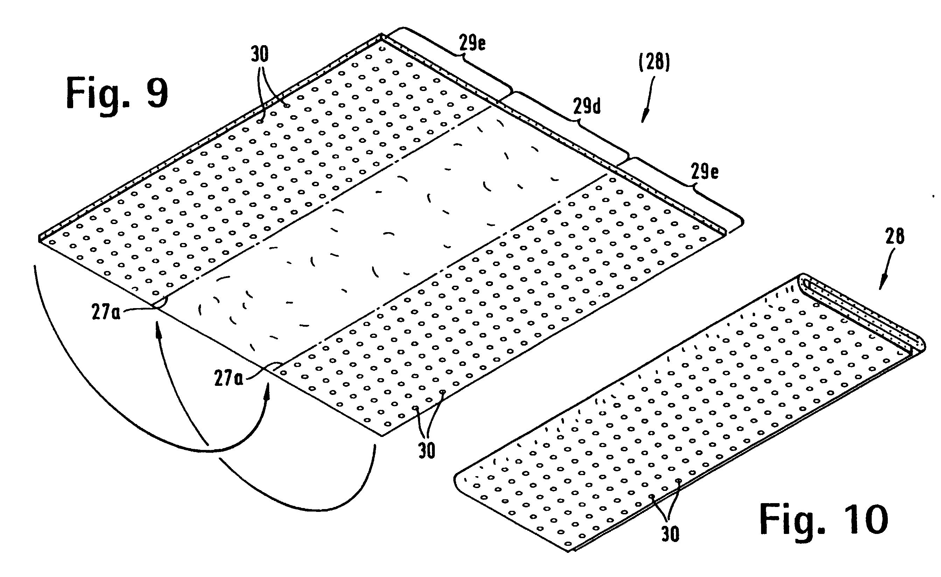

Fig. 9 zeigt eine andere Ausführungsform der weiteren Bahn 28. Diese weist ebenfalls, in Längsrichtung gesehen, eine zentrale, trichteröffnungsfreie Zone 29d auf. Die randseitig gelegenen Abschnitte 28c dagegen sind durchgehend mit Öffnungen 30 versehen. Bevor die weitere Bahn in dem erfindungsgemäßen absorbierenden Artikel Verwendung finden kann, wird diese entlang der Linien 27a gefaltet, wodurch die in Fig. 10 gezeigte Struktur erhalten wird. Auf den Bereich 29d kann die plissierte Bahn aufgebracht werden und die Bahn 28 mit den trichterförmigen Öffnungen 30 nach unten in den Artikel eingebaut werden.

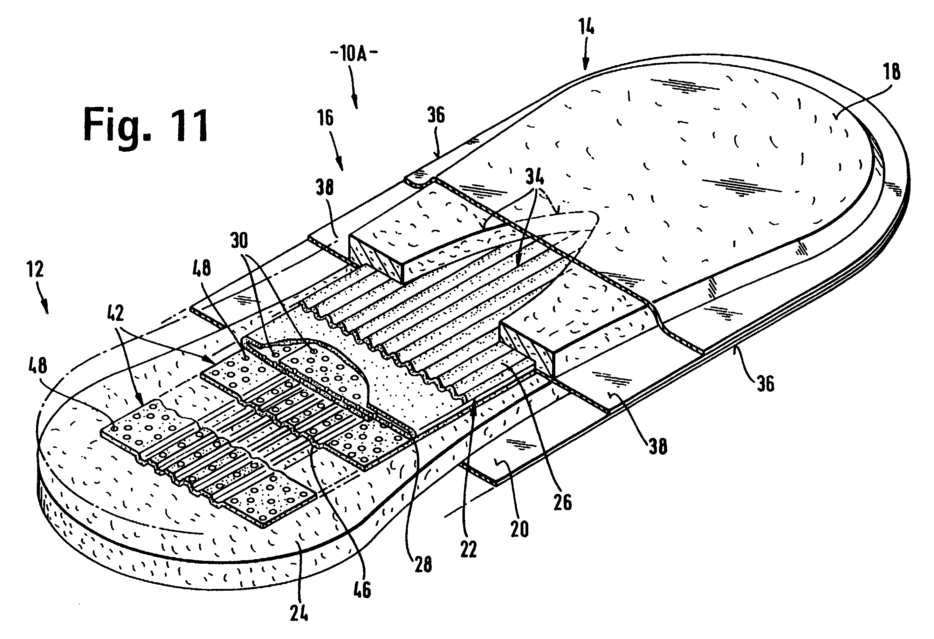

Fig. 11 zeigt schließlich eine weitere bevorzugte Ausgestaltung des erfindungsgemäßen absorbierenden Artikels.

Die Ausführungsform 10A gemäß Fig. 11 enthält als weitere Rückschlagsicherung die im wesentlichen flüssigkeitsundurchlässige bzw. halbdurchlässige Flüssigkeitssperrschicht 38. Diese verhindert, daß aus der Flüssigkeitsspeicherschicht 24 austretende Flüssigkeit in die flüssigkeitsaufnehmende Schicht 32 und von dort möglicherweise durch die flüssigkeitsdurchlässige Schicht 18 auf die Oberfläche des absorbierenden Artikels austreten kann. Die Sperrschicht 38 erstreckt sich dabei vom Rand 16 bis zur Flüssigkeitsverteilungsschicht 22. Die Schicht 38 kann ein integraler Bestandteil bzw. Fortsatz der flüssigkeitsundurchlässigen Schicht 20 sein und somit aus dem gleichen Material wie die Schicht 20 bestehen. Ein weiteres geeignetes Material für die Sperrschicht 38 ist Polyethylenoxid, was zu einer halbdurchlässigen Membran führt. Dieses kann als Schicht zwischen die flüssigkeitsdurchlässige Schicht 18 und die flüssigkeitsundurchlässige Schicht 20 eingefügt werden. Der Einbau einer undurchlässigen oder halbdurchlässigen Sperrschicht bietet sich immer dann an, wenn die Breite b der Flüssigkeitsverteilungsschicht kleiner ist als die geringste Breite B der Flüssigkeitsspeicherschicht 24 (siehe Fig. 1).

Weiterhin enthält der in Fig. 11 gezeigte absorbierende Artikel 10A eine zusätzliche Bahn 42, welche die längsgerichtete Flüssigkeitsverteilung in dem absorbierenden Artikel weiter unterstützt. Auch die Schicht 42 ist, wie die Schicht 28, aus einem UCTAD-Material hergestellt. Die Bahn 42 weist sich in Längsrichtung erstreckende Rillen 46 auf, welche die Flüssigkeitsverteilung in der gewünschten Längserstreckung des Artikels unterstützen. Des weiteren weist die Schicht 42 ebenfalls trichterförmige Öffnungen 48 auf, durch welche die Flüssigkeit in den Flüssigkeitsspeicher 24 übertreten kann.

## Patentansprüche

1. Absorbierender Artikel (10) mit
einem vorderen Bereich (12), einem hinteren Bereich (14) und einen zwischen vorderem und hinterem Bereich angeordneten Mittelteil (16),
einer oberen, bei Gebrauch des absorbierenden Artikels (10) dem Körper zugewandten, flüssigkeitsdurchlässigen Schicht (18),
einer unteren, bei Gebrauch des absorbierenden Artikels (10) dem Körper abgewandten, flüssigkeitsundurchlässigen Schicht (20),
einer zwischen der flüssigkeitsdurchlässigen Schicht (18) und der flüssigkeitsundurchlässigen Schicht (20) angeordneten Flüssigkeitsverteilungsschicht (22), welche sich vom vorderen Bereich (12) bis zum hinterem Bereich (14) des absorbierenden Artikels (10) erstreckt,
einer zwischen der flüssigkeitsundurchlässigen Schicht (20) und der Flüssigkeitsverteilungsschicht (22) angeordneten Flüssigkeitsspeicherschicht (24),
**dadurch gekennzeichnet daß**
die Flüssigkeitsverteilungsschicht (22) den direkten Durchtritt von Flüssigkeit in die Flüssigkeitsspeicherschicht (24) verhindert und einen gerichteten Transport der Flüssigkeit zu vorderen und/oder hinteren Bereichen (12, 14) des absorbierenden Artikels (10) bewirkt, um dadurch einen Flüssigkeitstransport längs der Flüssigkeitsverteilungsschicht (22) zu den vorderen und/oder hinteren Bereichen und danach in die Flüssigkeitsspeicherschicht (24) in den jeweiligen vorderen und/oder hinteren Bereichen zu erreichen, wodurch die Überführung der Flüssigkeit von der Flüssigkeitsverteilungsschicht (22) zu der Flüssigkeitsspeicherschicht (24) hauptsächlich in den jeweiligen vorderen und/oder hinteren Bereichen der Flüssigkeitsspeicherschicht erfolgt.

2. Absorbierender Artikel nach Anspruch 1, **dadurch gekennzeichnet, daß** die Flüssigkeitsverteilungsschicht (2) eine in Querrichtung des absorbierenden Artikels (10) gewellte Bahn (26), die in Längsrichtung Förderkanäle bildet, aufweist, und die gewellte Bahn (26) eine oberste Schicht (28a) in einer Flüssigkeitsverteilungsschicht mit mehreren Lagen in einer aufeinanderliegenden Beziehung ist.

3. Absorbierender Artikel (10) nach Anspruch 1 oder 2, wobei die Flüssigkeitsverteilungsschicht (22) eine weitere Bahn (28) enthält, die zumindest zwei Schichten (28b, 28c), aufweist, wobei die beiden Schichten (28b, 28c) trichterförmige Öffnungen (30) aufweisen, welche so ausgerichtet sind, daß sich die Trichteröffnungen in Richtung auf die Flüssigkeitsspeicherschicht (26) hin verjüngen.

4. Absorbierender Artikel (10) nach Anspruch 2 oder 3, wobei die gewellte Bahn (26) ein Vliesstoff ist.

5. Absorbierender Artikel (10) nach einem der Ansprüche 2 bis 4, wobei die gewellte Bahn (26) hochpigmentiert ist.

6. Absorbierender Artikel (10) nach einem der Ansprüche 3 bis 5, wobei die weitere Bahn (28) aus einem UCTAD-Material hergestellt ist.

7. Absorbierender Artikel (10) nach einem der vorhergehenden Ansprüche, wobei die Flüssigkeitsspeicherschicht (24) im vorderen Bereich (12) und/oder im hinteren Bereich (14) des absorbierenden Artikels ein höheres Flüssigkeitsrückhaltevermögen aufweist als im Mittelteil (16).

8. Absorbierender Artikel (10) nach einem der vorhergehenden Ansprüche, wobei Bereiche der Flüssigkeitsverteilungsschicht (22) und der Flüssigkeitsspeicherschicht (24) durch Pressen miteinander in Kontakt gebracht sind.

9. Absorbierender Artikel (10) nach Anspruch 8, wobei die Bereiche, welche durch Pressen miteinander in Kontakt gebracht sind, punktförmig ausgestaltet sind.

10. Absorbierender Artikel (10) nach einem der vorhergehenden Ansprüche, wobei die Flüssigkeitsverteilungsschicht (22) mit der Flüssigkeitsspeicherschicht (24) durch ein Haftmittel miteinander in Verbindung steht.

11. Absorbierender Artikel (10) nach Anspruch 10, wobei das Haftmittel ein hydrophiler Klebstoff ist.

12. Absorbierender Artikel (10) nach Anspruch 10 oder 11, wobei die Flüssigkeitsverteilungsschicht (22) und die Flüssigkeitsspeicherschicht (24) punktförmig durch das Haftmittel verbunden sind.

13. Absorbierender Artikel (10) nach Anspruch 10 oder 11, wobei die Flüssigkeitsverteilungsschicht (22) und die Flüssigkeitsspeicherschicht (24) durch ein geometrisches Muster miteinander in Verbindung stehen.

14. Absorbierender Artikel (10) nach Anspruch 13, wobei das geometrische Muster rautenförmig ausgestaltet ist.

15. Absorbierender Artikel (10) nach einem der Ansprüche 2 bis 14, wobei die trichterförmigen Öffnungen (30) in Randbereichen der weiteren Bahn (28) angeordnet sind und die Randbereiche so eingefaltet sind, daß sie unterhalb der gewellten Bahn (26) gelegen sind.

16. Absorbierender Artikel (10) nach Anspruch 15, wobei die in Längsrichtung verlaufenden Randbereiche derart umgeklappt sind, daß die sich verjüngenden Trichteröffnungen auf die Flüssigkeitsspeicherschicht (24) ausgerichtet sind.

17. Absorbierender Artikel (10) nach einem der Ansprüche 1 bis 16, wobei die trichterförmigen Öffnungen (30) an den sich verjüngenden Enden Saugfüßchen (40) aufweisen, welche mit der Flüssigkeitsspeicherschicht (24) in Eingriff stehen.

18. Absorbierender Artikel (10) nach einem der Ansprüche 2 bis 14, wobei die trichterförmigen Öffnungen (30) der Bahn (28) nur im vorderen Bereich (12) und/oder im hinteren Bereich (14) des absorbierenden Artikels (10) angeordnet sind.

19. Absorbierender Artikel (10) nach einem der vorhergehenden Ansprüche, wobei die Flüssigkeitsspeicherschicht (24) ein Zellstoffmaterial enthält.

20. Absorbierender Artikel (10) nach einem der vorhergehenden Ansprüche, welcher zwischen der flüssigkeitsdurchlässigen Schicht (18) und der Flüssigkeitsverteilungsschicht (22) eine flüssigkeitsaufnehmende Schicht (32) enthält.

21. Absorbierender Artikel (10) nach Anspruch 20, wobei die flüssigkeitsaufnehmende Schicht (32) eine zentral gelegene, durch die Schicht hindurchgehende Öffnung (34) aufweist.

22. Absorbierender Artikel (10) nach Anspruch 20 oder 21, wobei die flüssigkeitsaufnehmende Schicht (32) ein Zellstoffmaterial enthält.

23. Absorbierender Artikel (10) nach einem der vorhergehenden Ansprüche, welcher zusätzlich eine sich zwischen dem seitlichen Rand (36) des Artikels (10) und der Flüssigkeitsverteilungsschicht (22) erstreckende flüssigkeitsundurchlässige Sperrschicht (38) aufweist.

24. Absorbierender Artikel (10) nach einem der vorhergehenden Ansprüche, wobei die flüssigkeitsundurchlässige Schicht (20) und/oder die flüssigkeitsundurchlässige Sperrschicht (38) aus Polyethylen, Polypropylen oder Mischungen davon hergestellt sind.

25. Absorbierender Artikel (10) nach einem der vorhergehenden Ansprüche, wobei zwischen der Flüssigkeitsverteilungsschicht (22) und der Flüssigkeitsspeicherschicht (24) eine zusätzliche Bahn (42) angeordnet ist.

26. Absorbierender Artikel (10) nach Anspruch 25, wobei die zusätzliche Bahn (42) in Längsrichtung des Artikels verlaufende Rillen (46) aufweist.

27. Absorbierender Artikel (10) nach Anspruch 25 oder 26, wobei die zusätzliche Bahn (42) trichterförmige Öffnungen (48) enthält, deren sich verjüngende Trichteröffnungen mit der Flüssigkeitsspeicherschicht (24) in Kontakt stehen.

28. Absorbierender Artikel (10) nach einem der Ansprüche 25 bis 27, wobei die zusätzliche Bahn (42) aus einem UCTAD-Material hergestellt ist.

29. Absorbierender Artikel (10) nach einem der vorhergehenden Ansprüche, wobei auf der bei Gebrauch des Artikels körperabgewandten Seite der flüssigkeitsundurchlässigen Schicht (20) eine Haftschicht zur Befestigung des Artikels an einem Kleidungsstück angeordnet ist.

30. Absorbierender Artikel nach einem der vorhergehenden Ansprüche, wobei der absorbierende Artikel eine Damenbinde oder Damenhygieneeinlage ist.

## Claims

1. Absorbent article (10) including
a front area (12), a rear area (14), and a media area (16) arranged between the front and rear area,
an upper, liquid permeable layer (18) which, when the absorbent article (10) is in use, is turned toward the body,
a lower, liquid impermeable layer (20) which, when the absorbent article (10) is in use, is turned away from the body,
a liquid distribution layer (22) arranged between the liquid permeable layer (18) and the liquid impermeable layer (20) extending from the front area (12) to the rear area (14) of the absorbent article (10),
a liquid storage layer (24) arranged between the liquid impermeable layer (20) and the liquid distribution layer (22),
**characterized in that**
the liquid distribution layer (22) prevents the direct penetration of a liquid into the liquid storage layer (24) and leads to a directed conveyance of the liquid to the front and/or the rear areas (12, 14) of the absorbent article (10) in order to achieve in this manner a conveyance of liquid along the liquid distribution layer (22) to the front and/or rear areas and, subsequently, into the liquid storage layer (24) in the respective front and/or rear areas, thus causing the transfer of the liquid from the liquid distribution layer (22) to the liquid storage layer (24) primarily in the respective front and/or rear areas of the liquid storage layer.

2. An absorbent article according to claim 1, **characterized in that** the liquid distribution layer (2) is provided with a web (26), corrugated in the perpendicular direction of the absorbent article (10) and forming delivery channels in the longitudinal direction, and that the corrugated web (26) is located on the topmost layer (28a) of a liquid distribution layer including several stacked layers.

3. An absorbent article (10) according to claim 1 or 2, whereby the liquid distribution layer (22) includes another web (28), which is provided with at least two layers (28b, 28c), whereby the two layers (28b, 28c) are provided with funnel-shaped apertures (30) aligned such that the funnel apertures taper in the direction of the liquid storage layer (26).

4. An absorbent article (10) according to claim 2 or 3, in which the corrugated web (26) is a non-woven material.

5. An absorbent article (10) according to one of claims 2 to 4, whereby the corrugated web (26) is highly pigmented.

6. An absorbent article (10) according to one of claims 3 to 5, whereby the additional web (28) is manufactured from an UCTAD material.

7. An absorbent article (10) according to one of the preceding claims, whereby the liquid storage layer (24) in the front area (12) and/or in the rear area (14) of the absorbent article has a higher liquid retention capacity than in the media area (16).

8. An absorbent article (10) according to one of the preceding claims, whereby areas of the liquid distribution layer (22) and the liquid storage layer (24) are in contact with each other by means of pressure.

9. An absorbent article (10) according to claim 8, whereby the areas which are brought into contact with one another by means of pressure are designed in the form of points.

10. An absorbent article (10) according to one of the preceding claims, whereby the liquid distribution layer (22) is in contact with the liquid storage layer (24) by means of an adhesive.

11. An absorbent article (10) according to claim 10, whereby the adhesive is a hydrophilic adhesive.

12. An absorbent article (10) according to claim 10 or 11, whereby the liquid distribution layer (22) and the liquid storage layer (24) are connected in the form of points by the adhesive.

13. An absorbent article (10) according to claim 10 or 11, whereby the liquid distribution layer (22) and the liquid storage layer (24) are connected with each other by means of a geometrical pattern.

14. An absorbent article (10) according to claim 13, whereby the geometrical pattern is designed in the form of a rhombus.

15. An absorbent article (10) according to one of claims 2 to 14, whereby the funnel-shaped apertures (30) are arranged in the peripheral areas of the additional web (28), and the peripheral areas are folded inwards in such a way that they are positioned below the corrugated web (26).

16. An absorbent article (10) according to claim 15, whereby the peripheral areas running in the longitudinal direction are folded in such a way that the tapering funnel apertures are directed towards the liquid storage layer (24).

17. An absorbent article (10) according to one of claims 1 to 16, whereby the funnel-shaped apertures (30) are provided with suction feet (40) at the tapering ends, which engage with the liquid storage layer (24).

18. An absorbent article (10) according to one of claims 2 to 14, whereby the funnel-shaped apertures (30) of the web (28) are arranged only in the front area (12) and/or the rear area (14) of the absorbent article (10).

19. An absorbent article (10) according to one of the preceding claims, whereby the liquid storage layer (24) includes a cellulose material.

20. An absorbent article (10) according to one of the preceding claims, which contains a liquid absorbing layer (32) between the liquid permeable layer (18) and the liquid distribution layer (22).

21. An absorbent article (10) according to claim 20, whereby the liquid absorbing layer (32) is provided with a centrally located aperture (34) penetrating through said layer.

22. An absorbent article (10) according to claim 20 or 21, whereby the liquid absorbing layer (32) contains a cellulose material.

23. An absorbent article (10) according to one of the preceding claims, which is additionally provided with a liquid impermeable blocking layer (38) extending between the lateral edge (36) of the article (10) and the liquid distribution layer (22).

24. An absorbent article (10) according to one of the preceding claims, whereby the liquid impermeable layer (20) and/or the liquid impermeable blocking layer (38) are made of polyethylene, polypropylene, or mixtures thereof.

25. An absorbent article (10) according to one of the preceding claims, whereby an additional web (42) is arranged between the liquid distribution layer (22) and the liquid storage layer (24).

26. An absorbent article (10) according to claim 25, whereby the additional web (42) is provided with grooves (46) running in the longitudinal direction of the article.

27. An absorbent article (10) according to claim 25 or 26, whereby the additional web (42) includes funnel-shaped apertures (48), of which the tapering funnel apertures are in contact with the liquid storage layer (24).

28. An absorbent article (10) according to one of claims 25 to 27, whereby the additional web (42) is made of an UCTAD material.

29. An absorbent article (10) according to one of the preceding claims, whereby an adhesive layer for securing the article to a garment is provided on the side of the liquid impermeable layer (20) turned away from the body during use.

30. An absorbent article according to one of the preceding claims, whereby the absorbent article is a feminine napkin or a feminine hygiene insert.

## Revendications

1. Article absorbant (10) comprenant
une zone avant (12), une zone arrière (14), et une partie médiane (16) disposée entre la zone avant et la zone arrière,
une couche supérieure perméable aux fluides (18) et orientée vers le corps lors de l'utilisation de l'article absorbant (10),
une couche inférieure imperméable aux fluides (20), orientée à l'opposé du corps lors de l'utilisation de l'article absorbant (10),
une couche de répartition des fluides (22), disposée entre la couche perméable aux fluides (18) et la couche imperméable aux fluides (20), qui s'étend depuis la zone avant (12) jusqu'à la zone arrière (14) de l'article absorbant (10),
une couche de stockage des fluides (24) disposée entre la couche imperméable aux fluides (20) et la couche de répartition des fluides (22),
**caractérisé en ce que**
la couche de répartition des fluides (22) empêche la pénétration directe des fluides dans la couche de stockage des fluides (24) et génère un transport dirigé des fluides vers les zones avant et/ou arrière (12, 14) de l'article absorbant (10), afin de réaliser par ce biais une circulation des fluides le long de la couche de répartition des fluides (22) vers les zones avant et/ou arrière et ensuite dans la couche de stockage des fluides (24) des zones avant et/ou arrière respectives, le transfert des fluides de la couche de répartition des fluides (22) vers la couche de stockage des fluides (24) se faisant essentiellement dans les zones avant et/ou arrière respectives de la couche de stockage des fluides.

2. Article absorbant selon la revendication 1, **caractérisé en ce que** la couche de répartition des fluides (2) présente, dans la direction transversale de l'article absorbant (10), une feuille ondulée (26) qui forme dans la direction longitudinale des canaux de circulation, et **en ce que** la feuille ondulée (26) est une couche supérieure (28a) d'une couche de répartition des fluides comportant plusieurs couches selon une relation superposée.

3. Article absorbant (10) selon la revendication 1 ou 2, dans lequel la couche de répartition des fluides (22) comporte une autre feuille (28) qui présente au moins deux couches (28b, 28c), les deux couches (28b, 28c) présentant des ouvertures en forme d'entonnoir (30) qui sont orientées de façon telle que les ouvertures en entonnoir se rétrécissent dans la direction de la couche de stockage des fluides (26).

4. Article absorbant (10) selon la revendication 2 ou 3, dans lequel la feuille ondulée (26) est un non-tissé.

5. Article absorbant (10) selon l'une des revendications 2 à 4, dans lequel la feuille ondulée (26) est à pigmentation élevée.

6. Article absorbant (10) selon l'une des revendications 3 à 5, dans lequel l'autre feuille (28) est fabriquée à partir d'un matériau non crêpé séché par soufflage transversal.

7. Article absorbant (10) selon l'une des revendications précédentes, dans lequel la couche de stockage des fluides (24) présente dans la zone avant (12) et/ou dans la zone arrière (14) de l'article absorbant un pouvoir de rétention des fluides supérieur à celui de la partie médiane (16).

8. Article absorbant (10) selon l'une des revendications précédentes, dans lequel des zones de la couche de répartition des fluides (22) et de la couche de stockage des fluides (24) sont amenées en contact les unes avec les autres par gaufrage.

9. Article absorbant (10) selon la revendication 8, dans lequel les zones qui sont mises en contact les unes avec les autres par gaufrage sont réalisées sous forme de points.

10. Article absorbant (10) selon l'une des revendications précédentes, dans lequel la couche de répartition des fluides (22) est en relation avec la couche de stockage des fluides (24) par le biais d'un agent adhésif.

11. Article absorbant (10) selon la revendication 10, dans lequel l'agent adhésif est une colle hydrophile.

12. Article absorbant (10) selon la revendication 10 ou 11, dans lequel la couche de répartition des fluides (22) et la couche de stockage des fluides (24) sont reliées par l'agent adhésif au niveau de points.

13. Article absorbant (10) selon la revendication 10 ou 11, dans lequel la couche de répartition des fluides (22) et la couche de stockage des fluides (24) sont en relation l'une avec l'autre par le biais d'un motif géométrique.

14. Article absorbant (10) selon la revendication 13, dans lequel le motif géométrique revêt la forme de losanges.

15. Article absorbant (10) selon l'une des revendications 2 à 14, dans lequel les ouvertures en forme d'entonnoir (30) sont disposées dans les zones périphériques de l'autre feuille (28), et les zones périphériques sont repliées de façon telle qu'elles sont situées sous la feuille ondulée (26).

16. Article absorbant (10) selon la revendication 15, dans lequel les zones périphériques s'étendant dans la direction longitudinale sont rabattues de façon telle que les ouvertures en entonnoir sont orientées, côté rétréci vers la couche de stockage des fluides (24).

17. Article absorbant (10) selon l'une des revendications 1 à 16, dans lequel les ouvertures en forme d'entonnoir (30) présentent, au niveau des extrémités allant en se rétrécissant, des empattements d'absorption (40) qui viennent en prise avec la couche de stockage des fluides (24).

18. Article absorbant (10) selon l'une des revendications 2 à 14, dans lequel les ouvertures en forme d'entonnoir (30) de la feuille (28) sont uniquement disposées dans la zone avant (12) et/ ou dans la zone arrière (14) de l'article absorbant (10).

19. Article absorbant (10) selon l'une des revendications précédentes, dans lequel la couche de stockage des fluides (24) contient un matériau cellulosique.

20. Article absorbant (10) selon l'une des revendications précédentes, qui contient une couche d'absorption des fluides (32) entre la couche perméable aux fluides (18) et la couche de répartition des fluides (22).

21. Article absorbant (10) selon la revendication 20, dans lequel la couche d'absorption des fluides (32) présente une ouverture (34) placée de façon centrale et traversant la couche.

22. Article absorbant (10) selon la revendication 20 ou 21, dans lequel la couche d'absorption des fluides (32) contient un matériau cellulosique.

23. Article absorbant (10) selon l'une des revendications précédentes, qui présente en outre une couche barrière (38) imperméable aux fluides et s'étendant entre le bord latéral (36) de l'article (10) et la couche de répartition des fluides (22).

24. Article absorbant (10) selon l'une des revendications précédentes, dans lequel la couche imperméable aux fluides (20) et/ou la couche barrière imperméable aux fluides (38) est fabriquée à partir de polyéthylène, de polypropylène ou de mélanges de ceux-ci.

25. Article absorbant (10) selon l'une des revendications précédentes, dans lequel une feuille supplémentaire (42) est disposée entre la couche de répartition des fluides (22) et la couche de stockage des fluides (24).

26. Article absorbant (10) selon la revendication 25, dans lequel la feuille supplémentaire (42) présente des rainures (46) s'étendant dans la direction longitudinale de l'article.

27. Article absorbant (10) selon la revendication 25 ou 26, dans lequel la feuille supplémentaire (42) comporte des ouvertures en forme d'entonnoir (48), les ouvertures en forme d'entonnoir étant en contact, côté rétréci, avec la couche de stockage des fluides (24).

28. Article absorbant (10) selon l'une des revendications 25 à 27, dans lequel la feuille supplémentaire (42) est fabriquée à partir d'un matériau non crêpé, séché par soufflage transversal.

29. Article absorbant (10) selon l'une des revendications précédentes, dans lequel une couche adhésive est disposée sur le côté de la couche imperméable aux fluides (20) orienté à l'opposé du corps lors de l'utilisation de l'article, afin de fixer l'article à un vêtement.

30. Article absorbant selon l'une des revendications précédentes, dans lequel l'article absorbant est une serviette hygiénique ou un protège-slip.
